# EUROPEAN PATENT APPLICATION

(11) **EP 1 407 773 A1**
(43) Date of publication of application: **14.04.2004**
(21) Application number: 02022588.4
(22) Date of filing: 08.10.2002
(51) Int. Cl.: A61K 31/4422, A61P 9/10, C07D 211/90

(54) **A process for the preparation of s (-) amlodipine salts**

(71) Applicant: Council of Scientific and Industrial Research, New Delhi 110 001 (IN)
(72) Inventor: Rohini, Ramesh Joshi, Maharashtra (IN); Ramesh, Anna Joshi, Maharashtra (IN); Mukund, Keshav Gurjar, Maharashtra (IN)
(74) Representative: Winter, Brandl, Fürniss, Hübner, Röss, Kaiser, Polte Partnerschaft

(57) **Abstract**

Described herein is a process for the preparation of S(-)Amlodipine salts which comprises reaction of S(-)Amlodipine base with a solution of pharmaceutically acceptable acid such as benzene sulfonic acid, oxalic acid, maleic acid, succinic acid and p-toluene sulfonic acid. The reaction is carried out in the presence of an organic solvent at room temperature. The organic solvents include alcohols like ethanol, methanol, 2-propanol, hydrocarbons like toluene and polar solvents like dimethyl sulfoxide. The salt is obtained by addition of water and isolation of the salt formed by filtration. The unique feature of the invention is production of S(-) Amlodipine besylate in good chemical yield, high enantiomeric purity and with the quality required for preparation of pharmaceutical composition i.e. tablet formulation.

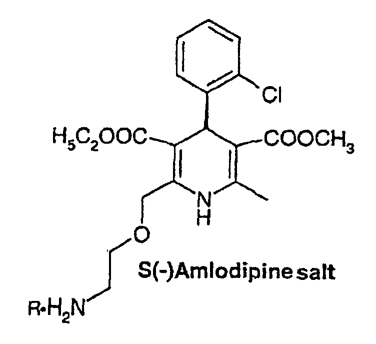

## Description

This invention relates to a process for the preparation of S(-) Amlodipine salts. More particularly it relates to a process for the preparation of pharmaceutically acceptable salt of S(-)Amlodipine selected from the group consisting of besylate, succinate, maleate, oxalate and tosylate. The S (-) Amlodipine salts of general formula (1) 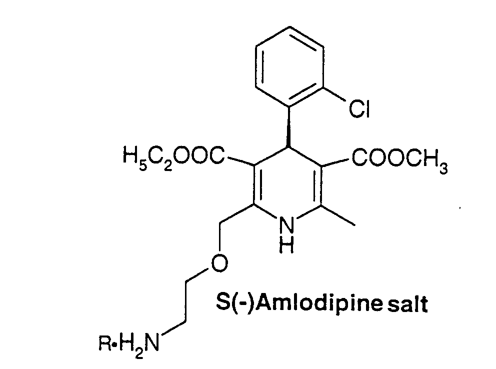 Wherein R is selected from the group consisting of benzene sulfonic acid, succinic acid, maleic acid, oxalic acid and p-toluene sulfonic acid.

These S(-)Amlodipine salts are prepared from S(-)Amlodipine, wherein S(- )Amlodipine is prepared by a procedure as described and claimed in our co-pending Indian patent application No. 262/Del/2002.

Of all the salts of S (-) Amlodipine mentioned above, the compound S (-) Amlodipine besylate; (4- S)-2-{[(2-aminoethyl)oxy]methyl}-4-(2-chlorophenyl)-6-methyl-1,4-dihydropyridine-3,5- dicarboxylate benzene sulfonate has commercial importance and is a potent and long acting calcium channel blocker.

(R,S)-Amlodipine besylate is currently being used for the treatment of cardiovascular disorders, in particular in the treatment of hypertension and angina, Amlodipine is racemic compound and has chiral center at 4 position of dihydropyridine ring. The S(-) isomer is having calcium channel blocker activity while the R(+)-isomer has little or no calcium channel blocking activity.

The compound R,S-Amlodipine is a potent and long acting calcium channel blocker having utility as an anti-ischaenic and anti-hypertensive agent. Although amlodipine is effective as the free base in practice it is best administered in the form of a salt of pharmaceutically acceptable acid, such as hydrochloride, hydrobromide, maleate, fumarate, tartarate and besylate.

Of these salts besylate is disclosed as being particularly preferred as it has good solubility, good stability, nonhygroscopicity and processability for tablet formation (- DE - 3710457, 1988, Edward Davison, James Ingram Wells).

In the prior art the preparation of R and S enantiomers of Amlodipine have been reported by resolution of amlodipine azide ester with optically active 2-methoxy-2-phenylethanol [**J. Med. Chem.,** 29, 1696, **1986.** J.E. Arrowsmith, S.F. Campbell, P.E.Cross, J.K. Stabs, R.A. Burges and EP Appl. 0331315A)] or resolution of Amlodipine base with optically active camphanic acid [**J. Med. Chem.**, 35, 3341, **1992**, S. Goldman, J. Stoltefuss and L. Born ] or resolution of RS-amlodipine base to R(+) and S(-) isomer with L or D-tartaric acid respectively in organic solvent DMSO, [ Peter L. Spargo **US patent** 6, 046,338; (2000), PCT 95/25722 (1995)] which indicate the use of both tartaric acids is essential.

Preparation of R and S amlodipine maleate salt has been reported starting from azido precursor. The procedure involves resolution of azido precursor using 2-methoxy-2-phenyl ethanol as a resolving agent, separation of diastereomer, ester exchange with sodium methoxide, hydrogenation, chromatographic purification and maleate salt formation. (J. Med. Chem, **29,** 1896, 1986. J.E. Arrowsmith, S.F. Campbell, P.E.Cross, J.K. Stabs, R.A. Burges).

Preparation of preferred amlodipine besylate salt has been disclosed in the publication (J. Chrom.B 693 (1997) 367-375 J. Luksa, Dj. Josic, B.Podobnik, B. Furlan, M. Kremser) describing the treatment of ethanolic solution of base with benzene sulfonic acid and isolation. The detailed procedures to obtain these salts have not been provided by the prior art. These prior art references also lack in providing physical or structural data given except the optical rotation except maleate.

The main object of the present invention therefore to provide a process for the preparation of S (-) Amlodipine salts

Another object is to provide the process to obtain the salts in good yields .

Yet another object is to obtain the salts with high enantiomeric purity.

Accordingly the present invention provides a process for the preparation of S (-) Amlodipine salts of formula (1) 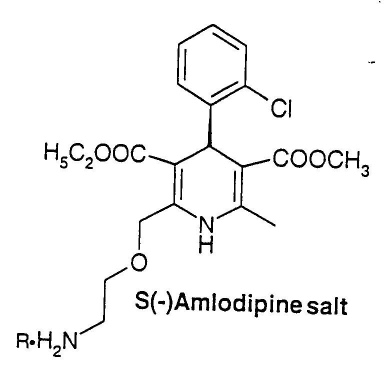 Wherein R is selected from the group consisting of benzene sulfonic acid, succinic acid, maleic acid, oxalic acid and p-toluene sulfonic acid, which comprises reacting S (-) amlodipine base with an aqueous solution of an acid with a molar ratio of S (-) amlodipine base to acid in the range of 1.2 : 1.0 to 1.0 : 1.2, in the presence of an organic solvent with a ratio of organic solvent to amlodipine in the range of 1.2 : 1.0 to 1.0 : 1.2 (v/w) under stirring at a temperature in the range of 20-300 °C, filtering the above said reaction mixture to obtain the solid mass and washing with cold water and hexane respectively followed by drying at a temperature of 35-450°C under vacuo to obtain the desired product in solid form.

In an embodiment of the present invention the organic solvent used is selected from the group consisting of alcohol, hydrocarbon and polar solvent.

In an another embodiment the organic solvent used is selected from the group consisting of dimethyl sulfoxide, 2-propanol, ethanol, methanol and toluene.

In yet another embodiment the acid used is selected from the group consisting of benzene sulfonic acid, succinic acid, maleic acid, oxalic acid and p-toluene sulfonic acid.

In still another embodiment the water to organic solvent ratio used is in the range of 5:1 to 8:1

The unique feature of the invention is production of S (-) amlodipine besylate with the quality required for preparation of pharmaceutical composition i.e. tablet formulation.

The process of the present invention is described herein below with reference to examples, which are illustrative only and should not be construed to limit the scope of the present invention in any manner.

### Example 1

### Amlodipine maleate from S (-) Amlodipine

Prepared by a procedure as described and claimed in our Indian co-pending patent application no. (262/Del/2002)

S (-) Amlodipine (5.0 gms, 0.012 moles, 98.2 ee) was dissolved in ethanol(10 ml) and maleic acid (1.42 gms 0.012 moles) in 70 ml of water was added with stirring. The separated solid was filtered washed with cold water, washed with hexane and dried under vacuo to give 5.32 gms(82.88%) of S(-) amlodipine maleate.mp.176-177oC Optical rotation [a]tD = -25.10 (c=1, MeOH) 98.31ee.

### Example 2

### Amlodipine oxalate from S (-) Amlodipine

S (-) Amlodipine (5.0 gms, 0.012 moles, 98.2 ee) was dissolved in ethanol(10 ml) and oxalic acid (1.54 gms 0.012 moles) in 70 ml of water was added with stirring. The separated solid was filtered washed with cold water, washed with hexane and dried under vacuo to give 5.80 gms (89.2%) of S(-) amlodipine oxalate. mp. 201-203oC Optical rotation [a]tD = -27.95 (c=1, MeOH) 98.41ee.

### Example 3

### Amlodipine succinate from S (-) Amlodipine

S (-) Amlodipine (5.0 gms, 0.012 moles, 98.2 ee) was dissolved in ethanol(10 ml) and succinic acid (1.44 gms 0.012 moles) in 70 ml of water was added with stirring. The separated solid was filtered washed with cold water, washed with hexane and dried under vacuo to give 6.0 gms(93.0%) of S(-) amlodipine succinate.mp.169-171o C Optical rotation [a]tD = -24.55 (c=1, MeOH) 97.95ee.

### Example 4

### Amlodipine tosylate from S (-) Amlodipine

S (-) Amlodipine (5.0 gms, 0.012 moles, 98.2 ee) was dissolved in ethanol (10 ml) and p-toluene sulfonic acid (2.32gms 0.012 moles) in 70 ml of water was added with stirring. The separated solid was filtered washed with cold water, washed with hexane and dried under vacuo to give 5.32 gms(82.88%) of S(-) amlodipine tosylate.mp.114-117o C Optical rotation [a]tD = - 20.2 (c=1, MeOH) 98.23ee.

### Example 5

### Amlodipine besylate from S (-) Amlodipine

S (-) Amlodipine (5.0 gms, 0.012 moles, 98.2 ee) was dissolved in ethanol(10 ml) and benzene sulfonic acid (1.93 gms 0.012 moles) in 70 ml of water was added with stirring. The separated solid was filtered washed with cold water, washed with hexane and dried under vacuo to give 5.32 gms(82.88%) of S(-) amlodipine besylate.mp.67-68 softens 107-108oC Optical rotation [a]tD = - 21.50 (c=1, MeOH) 98.15ee.

Microanalysis =C 50.91%, H 6.3%, N 4.67% S 5.91%: Calc for C20 H24O5N2Cl. C6H6O3S. 2.5 (H2O) C 51.1%, H 5.7%, N 4..58 % S 5.24%:

### Example 6

### a) S(-)Amlodipine-besylate from S(-)-Amlodipine

S(-) Amlodipine (62 gms, 0.152 moles, 93.1 ee) was dissolved in isopropanol (62 ml) and a solution of benzene sulfonic acid (24 gm, 0.152 moles) in 50 ml water was added maintaining the temperature ^{~}20°C. The reaction mixture was stirred for 30 min. and distilled water (450 ml) was added. The besylate salt separated after 20 min. stirring continued for one hr. and the slurry was filtered. Washed with distilled water, hexane. The solid was dried under vac. at 40°C. till constant wt. to give S(-) Amlodipine besylate (83 gm, 89% yield) 93.3 ee

### b) Recrystallisation of S(-) Amlodipine besylate

S(-) Amlodipine besylate (80 gms., 93.1 ee) was dissolved in isopropanol (80 ml) The reaction mixture was stirred for 30 min. and distilled water (640 ml) was added.

The besylate salt separated after 20 min. stirring continued for one hr. and the slurry was filtered. Washed with distilled water, hexane. The solid was dried under vacuo at 40°C. till constant wt. to give S(-) Amlodipine besylate (63 gm, 98.43 ee).

### Example 7

### S(-)Amlodipine-besylate from S(-)-Amlodipine

S(-) Amlodipine (62 gms, 0.152 moles, 98.2 ee) was dissolved in isopropanol (62 ml) and a solution of benzene sulfonic acid (24 gm, 0.152 moles) in 50 ml water was added maintaining the temperature ^{~}20°C. The reaction mixture was stirred for 30 min. and distilled water (450 ml) was added.

The besylate salt separated after 20 min. stirring continued for one hr and the slurry was filtered. Washed with distilled water, hexane. The solid was dried under vacuo at 40°C. till constant wt. to give S(-) Amlodipine besylate (83 gm, 89% yield) 98.3 ee

Optical purity (enantiomeric excess ee) was determined using Chiral HPLC. Ultron chiral chrompak column 15 cm; Flow rate-1ml/min; Detection wavelength 360 nm;

Mobile phase Disodium hydrogen phosphate buffer (pH6.9):acetonitrile, 80:20.

Rt - R=6.1 min., S=7.3 min.

### Advantages of the present invention are as follows:

The Process describes for the first time in detail the preparation of S(-)Amlodipine besylate salt in good chemical yields, high enantiomeric purity and with the quality required for preparation of pharmaceutical composition i.e. tablet formulation.

## Claims

1. A process for the preparation of S (-) Amlodipine salts of formula (1) 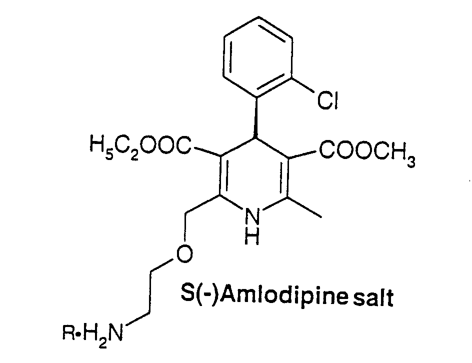 wherein R is selected from the group consisting of benzene sulfonic acid, succinic acid, maleic acid, oxalic acid and p-toluene sulfonic acid, which comprises reacting S (-) amlodipine base with an aqueous solution of an acid with a molar ratio of S (-) amlodipine base to acid in the range of 1.2 : 1.0 to 1.0 : 1.2, in the presence of an organic solvent with a ratio of organic solvent to amlodipine in the range of 1.2 : 1.0 to 1.0 : 1.2 (v/w) under stirring at a temperature in the range of 20-300 °C, filtering the above said reaction mixture to obtain the solid mass and washing with cold water and hexane respectively followed by drying at a temperature of 35-450°C under vacuo to obtain the desired product in solid form.

2. A process as claimed in claim 1, wherein the organic solvent used is selected from the group consisting of alcohol, hydrocarbon and polar solvent.

3. A process as claimed in claims 1&2, wherein the organic solvent used is selected from the group consisting of dimethyl sulfoxide, 2-propanol, ethanol, methanol and toluene.

4. A process as claimed in claims 1-3, wherein the acid used is selected from the group consisting of benzene sulfonic acid, succinic acid, maleic acid, oxalic acid and p- toluene sulfonic acid.

5. A process as claimed in claims 1-4, wherein water to organic solvent ratio used is about 8:1.

6. A process for the preparation of S (-) Amlodipine salts of formula (1), substantially as herein described with reference to the examples.
